# EUROPÄISCHE PATENTSCHRIFT

(11) **EP 1 051 951 B1**
(45) Veröffentlichungstag und Bekanntmachung des Hinweises auf die Patenterteilung: **30.11.2005**
(21) Anmeldenummer: 00890148.0
(22) Anmeldetag: 10.05.2000
(51) Int. Cl.: A61C 8/00

(54) **Zahnimplantat**
Dental implant
Implant dentaire

(30) Priorität: 10.05.1999 AT 84499
(43) Veröffentlichungstag der Anmeldung: 15.11.2000
(73) Patentinhaber: MKE Metall- und Kunststoffwaren Erzeugungsgesellschaft m.b.H., 3860 Heidenreichstein (AT)
(72) Erfinder: Grabenhofer, Andreas, 3873 Brand (AT)
(74) Vertreter: Müllner, Erwin

(56) Entgegenhaltungen:
- EP-A- 0 748 616
- US-A- 5 695 335

## Beschreibung

Die vorliegende Erfindung betrifft eine Zahnimplantat mit einem im Kieferknochen zu verankernden Ennosalteil, der aus einem oberen und einem unteren Abschnitt besteht, wobei diese beiden Abschnitte in Axialrichtung auseinander bewegbar sind, wobei der untere Abschnitt einen rohrförmigen Fortsatz aufweist, der in eine entsprechende zylindrische Ausnehmung des oberen Abschnittes ragt, wobei weiters der rohrförmige Fortsatz ein Innengewinde aufweist, in das eine Schraube zur Fixierung eines Implantatkopfes eindrehbar ist.

Die Anmelderin führt seit einiger Zeit Versuche mit derartigen Zahnimplantaten durch. Der Sinne dieser Zahnimplantate besteht darin, dass dasselbe Implantat, das später die Suprakonstruktion tragen soll, zunächst zur Hebung eines geschwundenen Kieferknochens eingesetzt wird.

Diese Zahnimplantate werden wie folgt verwendet: Man schneidet in den Kieferknochen einen horizontalen Schlitz. Dann bohrt man in bekannter Weise an den beiden Enden des Schlitzes (aber noch innerhalb des Schlitzes) zwei Löcher für zwei Zahnimplantate. In diese setzt man den erfindungsgemäßen Enossalteil so ein, dass die Trennfläche der beiden Abschnitte im Schlitz des Kieferknochens liegt. Danach schneidet man noch zwei vertikale Schlitze in den Kiefer, und zwar außerhalb der beiden Enossalteile und bis zu dem horizontalen Schlitz. Damit ist nun ein Knochensegment völlig vom restlichen Kiefer getrennt. Er ist nur durch die oberen Abschnitte der Enossalteile fixiert. Nachdem die beiden Enossalteile eingeheilt sind (nach etwa einer Woche), wird der obere Abschnitt des Enossalteils vom unteren Abschnitt entfernt, und zwar ganz langsam (etwa 1/10 mm bis 1/mm pro Tag). Auf diese Weise wird das abgetrennte Knochensegment vom ursprünglichen Knochen entfernt, sodass im horizontalen Schlitz das Knochenwachstum angeregt wird. Nachdem genügend Knochensubstanz gebildet ist, wird auf den Enossalteil - ähnlich wie bei bekannten Implantaten - ein Implantatkopf aufgeschraubt und darauf die Suprakonstruktion befestigt.

Solch ein Zahnimplantat ist aber auch für den Ersatz von Einzelzähnen geeignet. In diesem Fall führt man den horizontalen Schlitz nur geringfügig breiter aus als einen Zahn und hebt das abgetrennte Stück mit nur einem Zahnimplantat.

Bei den von der Anmelderin durchgeführten Versuchen hat sich herausgestellt, dass beim fertigen Zahnimplantat bestehend aus Suprakonstruktion, Implantatkopf, oberem Abschnitt und unterem Abschnitt eine Schwachstelle vorhanden ist, nämlich der rohrförmige Fortsatz des unteren Abschnittes, der aus geometrischen Gründen relativ dünnwandig ausgeführt sein muss. Ein weiterer Nachteil besteht darin, dass schräge Kräfte auf die Suprakonstruktion vom Implantatkopf nicht direkt auf den unteren Abschnitt übertragen konnten, sondern nur auf den oberen Abschnitt.

Es ist Aufgabe der vorliegenden Erfindung, das Zahnimplantat der eingangs genannten Art stabiler zu machen.

Diese Aufgabe wird gemäß der vorliegenden Erfindung bei einem Zahnimplantat der eingangs genannten Art dadurch gelöst, dass der Implantatkopf einen zylindrischen Fortsatz aufweist, der in den rohrförmigen Fortsatz des unteren Abschnittes eingreift. Dadurch, dass der zylindrische Fortsatz des Implantatkopfes in den rohrförmigen Fortsatz des unteren Abschnittes eingreift, erfolgt eine direkte Einleitung schräger Kräfte vom Implantatkopf in den unteren Abschnitt.

Es ist zweckmäßig, wenn der rohrförmige Fortsatz eine Ausnehmung für den zylindrischen Fortsatz aufweist und wenn der Innendurchmesser des rohrförmigen Fortsatzes etwa dem Innendurchmesser des zylindrischen Fortsatzes entspricht. Durch den etwa gleichen Innendurchmesser des zylindrischen Fortsatzes des Implantatkopfes und des rohrförmigen Fortsatzes des unteren Abschnittes liegt die Schraube zur Fixierung des Implantatkopfes auch im zylindrischen Fortsatz innen an, wodurch die Stabilität für schräge Kräfte weiter erhöht wird.

Besonders vorteilhaft ist es, wenn sich die Schraube zumindest durch den gesamten rohrförmigen Abschnitt hindurch erstreckt. Auf diese Weise wird der rohrförmige Abschnitt durch die Schraube stabilisiert. Die Schraube kann dabei bis in dem Bereich ragen, mit dem der untere Abschnitt im Kieferknochen eingeschraubt ist und der entsprechend dick und stabil ausgeführt ist. Somit können schräge Kräfte, die auf die Suprakonstruktion und somit auf den Implantatkopf wirken, direkt bis in den Bereich des unteren Abschnittes eingeleitet werden, der mit dem Kieferknochen verschraubt ist.

Nach einer weiteren Ausführungsform der vorliegenden Erfindung ist vorgesehen, dass der obere Anschnitt und der Implantatkopf jeweils eine ebene Fläche aufweisen, die beim Einschrauben des Implantatkopfes in den oberen Abschnitt in kraftschlüssige Verbindung treten.

Der Implantatkopf dient zur Aufnahme der prothetischen Versorgungselemente. Auf diesen Implantatkopf werden somit jene Teile montiert, die sichtbar in die Mundhöhle ragen und den verlorenen Zahn bzw. die verlorenen Zähne ersetzen. Der Aufbau von für diese Zwecke eingesetzten prothetischen Versorgungen ist allgemein bekannt. Der Implantatkopf kann im oberen Ende so abgestimmt sein, dass die von der Anmelderin bzw. Patentinhaberin hergestellten Standardaufbauelemente weiterhin verwendet werden können. Durch die ebenen Flächen des oberen Abschnittes und des Implantatkopfes kann eine Verringerung der Höhe der Bauform erreicht werden, sodass eine optimale Anpassung an die anatomischen Gegebenheiten möglich und somit der untere Kronenrand so weit wie möglich in Richtung Knocheneintrittsebene verlagert ist.

Besonders günstig ist es, wenn der obere Abschnitt eine ebene Fläche aufweist und der Implantatkopf eine leicht konische Fläche, sodass beim Einschrauben des Implantatkopfes in den oberen Abschnitt der äußere Rand der konischen Fläche mit hohem Druck auf der ebenen Fläche aufliegt. Auf diese Weise wird zusätzlich zu der Verringerung der Höhe der Bauform erreicht, dass eine Abdichtung gegen möglicherweise eintretende Gewebeflüssigkeit gegeben ist. Es entsteht eine Art anpressender Dichtring, der zudem die Fügefestigkeit zwischen diesen beiden Teilen durch die beim Einschrauben entstehende Friktion erhöht.

Während der klinischen Versuche mit Zahnimplantaten hat sich gezeigt, dass in der Distraktionsphase ein minimales Absinken des vom ursprünglichen Knochen getrennten Knochensegmentes möglich sein kann. Dieses Absinken kann sich mitunter negativ auf den Heilungserfolg auswirken. Es ist daher zweckmäßig, wenn der obere Abschnitt einen teilweise offenen Ring, in dem eine Schraube zur Befestigung des Ringes am oberen Abschnitt eingeschraubt ist, aufweist. Der Ring ist teilweise offen gestaltet, damit er im Bereich des rohrförmigen Fortsatzes des unteren Abschnittes, der im oberen Abschnitt verschiebbar gelagert ist, eingeschoben werden kann und nach der Insertion mit Hilfe der im Ring gelagerten Schraube am oberen Abschnitt fixiert werden kann.

Es ist zweckmäßig, wenn die Fläche des oberen Abschnittes zur Befestigung des Ringes konisch ausgeformt ist. Dadurch schiebt sich die im Ring gelagerte Schraube entlang dieser konischen Fläche nach oben und presst somit das mit dem oberen Abschnitt verbundene Knochensegment fest an diesen an. Weil die Schnittführung während der Trennung des Knochensegmentes nicht exakt parallel zur Abschlussfläche des oberen Abschnittes erfolgen kann, ist nach einem weiteren Merkmal der Erfindung vorgesehen, dass der teilweise offene Ring im Bereich seiner Bohrung konvex ausgeführt ist, sodass eine Ausrichtung am chirurgisch getrennten Knochensegment möglich ist. Somit kann sich der Ring bedingt durch seine Bohrungsführung und durch die konische Fläche am oberen Abschnitt so auspendeln, dass er möglichst optimal an dem getrennten Knochensegment anliegt. Dies wird dadurch erreicht, dass nach der vollständigen Trennung des Knochensegmentes eine Probedistraktion durchgeführt wird, um einerseits sicherzustellen, dass dieses Segment vollständig getrennt wurde, und um anderseits den teilweise offenen Ring mit Hilfe eines dafür vorgesehenen Instrumentes positionieren zu können. Der Ring wird zunächst in das Instrument gelegt und danach mit dem Instrument eingebracht. Sobald der Ring eingebracht wurde, wird das Implantat wieder zusammengeschraubt, wodurch der im Instrument liegende Ring zwischen ursprünglichem Knochen und getrenntem Knochensegment eingeklemmt wird und sich an der Schnittebene ausrichtet. In dieser Lage wird nun die im Ring gelagerte Schraube fixiert, wodurch ein optimales Anliegen des Ringes am getrennten Knochensegment gewährleistet ist. Nach dieser Fixierung des Ringes wird das Zahnimplantat wieder so weit auseinander geschoben, bis das Instrument zur Ringinsertion entfernt werden kann. Abschließend wird das Zahnimplantat in die zur Distraktion erforderliche Ausgangslage zurückgeschoben, und der zur Einheilung erforderliche Wundverschluss beendet den chirurgischen Eingriff.

Schließlich ist es zweckmäßig, wenn der zur Lagefixierung vorgesehene Implantatkopf in Bezug auf seine Formgebung im oberen Ende an die zur prothetischen Versorgung erforderlichen Befestigungsteile angepasst ist. Man kann also jeweils eine einheitliche Bauform für den oberen und den unteren Abschnitt des Zahnimplantates wählen und nur den Implantatkopf jeweils so ausbilden, dass er optimal an die gewählte Suprakonstruktion angepasst ist.

Um ein Einwachsen von Gewebe in den unteren Abschnitt zu vermeiden, ist es zweckmäßig, wenn die Bohrung zur Aufnahme der Schraube im unteren Abschnitt eine Sackbohrung ist, oder - wenn sie eine Durchgangsbohrung ist - dann soll am Ende ein Stopfen eingepresst oder eingeschweißt sein. Ein eingeschraubter Stopfen kann in der Regel das Einwachsen von Gewebe nicht mit Sicherheit verhindern.

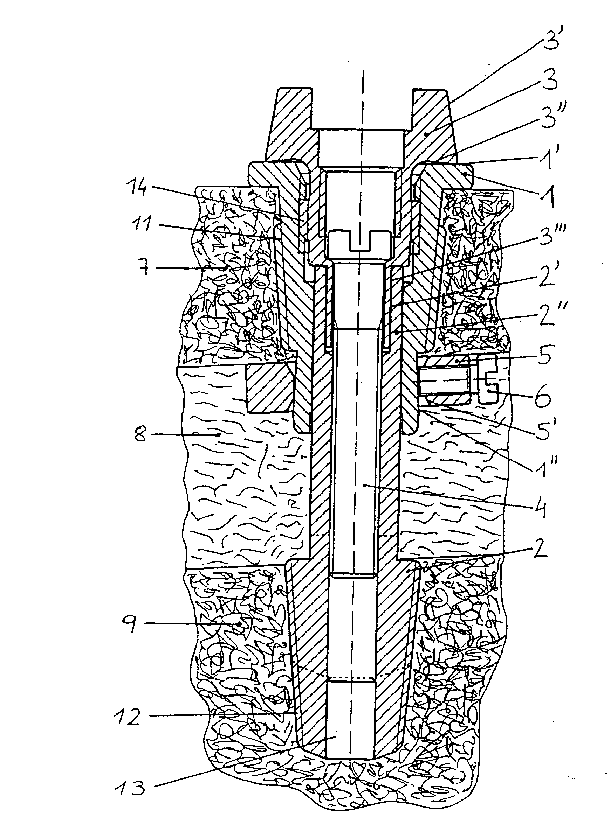

Anhand der beiliegenden Zeichnung wird vorliegende Erfindung näher erläutert. Die einzige Figur zeigt ein erfindungsgemäßes Zahnimplantat im Schnitt, eingesetzt in ein bereits distrahiertes Kiefer.

Das Zahnimplantat weist einen Ennosalteil auf, der aus einem oberen Abschnitt 1 und einem unteren Abschnitt 2 zusammengesetzt ist. Beide Abschnitte 1 und 2 weisen ein Außengewinde 11 bzw. 12 auf, deren Gewindesteigung gleich ist, damit sie gemeinsam in den Kiefer eingeschraubt werden können. Vom Kiefer 9 ist ein Knochensegment 7 abgetrennt worden, dazwischen hat sich bereits Knochen 8 neu gebildet. Die beiden Außengewinde 11 bzw. 12 sind etwas kegelförmig dargestellt, sie können aber auch exakt zylindrisch ausgeführt werden. Es kann auch die Gewindeform variiert werden.

Der untere Abschnitt 2 weist einen rohrförmigen Fortsatz 2" auf, der sich in eine entsprechende zentrische Ausnehmung des oberen Abschnittes 1 hinein erstreckt. Der rohrförmige Fortsatz 2" weist ein Innengewinde zur Aufnahme der Schraube 4 auf.

In den oberen Abschnitt 1 ist über eine Gewindeverbindung 14 ein Kopfteil 3 eingeschraubt. Er liegt mit einer leicht konischen Fläche 3" an einer ebenen Fläche 1' des oberen Abschnittes 1 an. Auf diese Weise entsteht eine flüssigkeitsdichte Verbindung zwischen dem Implantatkopf 3 und dem oberen Abschnitt 1, weil die äußere Kante der leicht konischen Fläche 3" durch das Einschrauben mit hohem Druck an die ebene Fläche 1' angepresst wird. Das Einwachsen von Körpergewebe wird dadurch unterbunden. Vorteilhaft ist weiters, dass der obere Abschnitt 1 den Kieferknochen 7 nur minimal überragt: die ebene Fläche 1' liegt nur um die notwendige Materialstärke über dem Kieferknochen 7. Es ist daher äußerst unwahrscheinlich, dass der aus Titan bestehende Ennosalteil sichtbar wird, wenn das Implantat fertig eingeheilt ist.

Der Implantatkopf 3 ist nicht nur über die Gewindeverbindung 14 mit dem oberen Abschnitt 1 verbunden, sondern zusätzlich auch mit dem unteren Abschnitt 2. Zu diesem Zweck weist der Implantatkopf 3 einen zylindrischen Fortsatz 3"' auf, der in eine Ausnehmung 2' des rohrförmigen Fortsatzes 2" des unteren Abschnittes 2 hineinragt. Außerdem ist der Implantatkopf 3 mittels der Schraube 4, welche in den rohrförmigen Fortsatz 2" eingeschraubt ist, mit dem unteren Abschnitt 2 verbunden. Die Schraube 4 erstreckt sich dabei durch den gesamten rohrförmigen Fortsatz 2" hindurch bis in jenen Bereich des unteren Abschnittes 2, der das Gewinde 12 aufweist. Die Schraube 4 füllt somit den gesamten rohrförmigen Fortsatz aus, sodass dieser eine hohe Knickfestigkeit aufweist.

Damit nicht das abgetrennte Knochensegment 7 absinken kann bzw. um das Gewinde 11 zu entlasten, ist ein Ring 5 vorgesehen, der teilweise offen ist. Die Öffnung muss so groß sein, dass der Ring von außen über den rohrförmigen Fortsatz 2' geschoben werden kann. Der Ring 5 wird dann angehoben, bis er - wie in der Figur gezeigt - im Bereich einer konischen Fläche 1" des oberen Abschnittes 1 liegt. Der Ring 5 ist im Bereich seiner Bohrung 5' mit einer konvexen Oberfläche versehen. Diese kann z.B. dadurch hergestellt sein, dass die Oberfläche im Bereich der Bohrung 5' einen mittleren, zylindrischen Abschnitt, und daran zu beiden Seiten anschließend jeweils einen kegelförmigen Abschnitt aufweist. Der Ring 5 weist eine Gewindebohrung auf, in die eine Schraube 6 eingeschraubt werden kann. Diese Schraube 6 dient zur Fixierung des Ringes 5. Die konische Fläche 1" bewirkt, dass der Ring 5 selbst dann, wenn sich die Schraube 6 geringfügig lockern sollte, nicht nennenswert nach unten verschieben lässt. Die konische Fläche 1" bewirkt mit der konvexen Fläche des Ringes 5, dass dieser nicht unbedingt genau normal zur Längsachse des oberen Abschnittes 1 stehen muss. Im Allgemeinen gelingt es nämlich nicht, die Trennfläche zwischen dem abgetrennten Knochensegment 7 und dem ursprünglichen Knochen 9 genau normal zur Bohrung für den Ennosalteil auszuführen. Bei der dargestellten Konstruktion passt sich die Lage des Ringes 5 dieser Ungenauigkeit an.

Der erfindungsgemäße Ennosalteil wird wie folgt verwendet: zunächst wird in den Kiefer ein horizontaler Schlitz gesägt, der das Knochensegment 7 vom restlichen Knochen 9 trennt. (Dieser Schlitz wird dann später vom neu gebildeten Knochen 8 ausgefüllt.) Danach wird für zwei erfindungsgemäße Zahnimplantate jeweils eine Bohrung (Implantatbett) mit einem speziell dafür abgestimmten Bohrersystem ausgeführt. In jedes Implantatbett wird ein erfindungsgemäßes Zahnimplantat eingeschraubt, wobei aber die beiden Abschnitte 1 und 2 so weit wie möglich zusammengeschoben sind. Um dies zu erleichtern, kann das obere Ende des oberen Abschnittes 1 einen mehrkantigen Anschlussrand aufweisen, sodass ein entsprechendes Werkzeug angesetzt werden kann. Außerdem muss eine Verdrehsicherung zwischen dem oberen Abschnitt 1 und dem unteren Abschnitt 2 vorgesehen sein, die jedenfalls dann wirksam ist, wenn die beiden Abschnitte 1 und 2 so weit wie möglich zusammengeschoben sind. Auf diese Weise lassen sich dann die beiden Abschnitte 1 und 2 gemeinsam in das Implantatbett einschrauben. Danach werden vertikale Schlitze außerhalb der beiden Implantate gesägt, sodass das Knochensegment 7 nun vollkommen vom ursprünglichen Knochen 9 getrennt ist und nur von den Gewinden 11 der oberen Abschnitte 1 gehalten wird.

Damit die beiden Abschnitte 1 und 2 definiert auseinander bewegt werden können, ist anstelle des Implantatkopfes 3 ein Kopfteil vorgesehen, in dem eine Schraube drehbar, aber axial unverschiebbar gelagert ist. Diese Schraube ist in das Innengewinde des rohrförmigen Fortsatzes 2" eingeschraubt. Durch Herausdrehen der Schraube vergrößert sich der Abstand zwischen dem oberen Abschnitt 1 und dem unteren Abschnitt 2.

Nach Durchführung der beiden vertikalen Schnitte wird eine Probedistraktion durchgeführt, indem die Schrauben in den beiden Implantaten gedreht werden. Dadurch wird sichergestellt, dass das Knochensegment 7 vollständig abgetrennt wurde. Anderseits ist diese Probedistraktion auch notwendig, um den Ring 5 positionieren zu können. Der Ring 5 wird mit einem speziell dafür vorgesehenen Instrument eingebracht, und zwar im Bereich des freiliegenden rohrförmigen Fortsatzes 2", weil dies die dünnste Stelle das Zahnimplantates ist. Dann wird das Implantat wieder zusammengeschraubt, wodurch der im Instrument liegende Ring 5 zwischen dem ursprünglichen Knochen 9 und dem abgetrennten Knochensegment 7 eingeklemmt wird, sodass er sich automatisch an der Schnittebene ausrichtet. In dieser Lage wird nun die Schraube 6 fixiert, wodurch ein optimales Anliegen des Ringes am Knochensegment 7 gewährleistet ist. Nun muss das Zahnimplantat nochmals so weit auseinander geschoben werden, dass das Instrument entfernt werden kann. Abschließend wird das Zahnimplantat in die Ausgangslage zurückgeschoben und der zur Einheilung erforderliche Wundverschluss beendet den chirurgischen Eingriff. Nach Abwarten einer primären Einheilphase wird täglich die Schraube gedreht, sodass der obere Abschnitt 1 vom unteren Abschnitt 2 weg bewegt wird. Dadurch wird auch das Knochensegment 7 vom ursprünglichen Knochen 9 weg bewegt, sodass im Spalt das Knochenwachstum angeregt wird (neu gebildeter Knochen 8). Diese kontinuierliche Verschiebung erfolgt in Form von geringen täglichen Bewegungen in der Größenordnung von ca. 0,1 bis 1,0 mm.

Nach Beendigung der Distraktion werden die Schraube und der Kopfteil entfernt und gegen den Implantatkopf 3 ausgetauscht, der zunächst in den oberen Abschnitt 1 eingeschraubt wird und dann mit der Schraube 4 zusätzlich im unteren Abschnitt 2 fixiert wird. Auf diesen Implantatkopf 3 wird dann die Suprakonstruktion befestigt. Es ist dabei zweckmäßig, jeweils einen solchen Implantatkopf auszuwählen, dessen oberes Ende 3' eine Form hat, die sich optimal mit der gewählten Suprakonstruktion verbinden lässt.

Die Bohrung zur Aufnahme der Schraube 4 ist in der Zeichnung als Durchgangsbohrung dargestellt. Um ein Einwachsen von Gewebe zu verhindern, ist diese Bohrung mit einem Stopfen 13 verschlossen. Um einen dichten Abschluss zu bewirken, kann der Stopfen 13 mit einem Presssitz in der Durchgangsbohrung anliegen; es ist aber auch möglich, den Stopfen 13 mittels Laser einzuschweißen, um so einen dichten Abschluss zu bewirken. Selbstverständlich kann auch anstelle einer Durchgangsbohrung eine Sachbohrung ausgeführt werden.

## Patentansprüche

1. Zahnimplantat mit einem Implantatkopf und mit einem im Kieferknochen zu verankernden Ennosalteil, der aus einem oberen (1) und einem unteren (2) Abschnitt besteht, wobei diese beiden Abschnitte (1, 2) in Axialrichtung auseinander bewegbar sind, wobei der untere Abschnitt (2) einen rohrförmigen Fortsatz (2") aufweist, der in eine entsprechende zylindrische Ausnehmung des oberen Abschnittes (1) ragt, wobei weiters der rohrförmige Fortsatz (2") ein Innengewinde aufweist, in das eine Schraube (4) zur Fixierung des Implantatkopfes (3) eindrehbar ist, **dadurch gekennzeichnet, dass** der Implantatkopf (3) einen zylindrischen Fortsatz (3"') aufweist, der in den rohrförmigen Fortsatz (2") des unteren Abschnittes (2) eingreift.

2. Zahnimplantat nach Anspruch 1, **dadurch gekennzeichnet, dass** der rohrförmige Fortsatz (2") eine Ausnehmung (2') für den zylindrischen Fortsatz (3"') aufweist und dass der Innendurchmesser des rohrförmigen Fortsatzes (2") etwa dem Innendurchmesser des zylindrischen Fortsatzes (3"') entspricht.

3. Zahnimplantat nach Anspruch 1 oder 2, **dadurch gekennzeichnet, dass** sich eine Schraube (4) zumindest durch den gesamten rohrförmigen Abschnitt (2") hindurch erstreckt.

4. Zahnimplantat nach einem der Ansprüche 1 bis 3, **dadurch gekennzeichnet, dass** der obere Abschnitt (1) und er Implantatkopf (3) jeweils eine ebene Fläche (3", 1') aufweisen, die beim Einschrauben des Implantatkopfes (3) in den oberen Abschnitt (1) in kraftschlüssige Verbindung treten.

5. Zahnimplantat nach einem der Ansprüche 1 bis 3, **dadurch gekennzeichnet, dass** der obere Abschnitt (1) eine ebene Fläche (1') aufweist und der Implantatkopf (3) eine leicht konische Fläche (3"), sodass beim Einschrauben des Implantatkopfes (3) in den oberen Abschnitt (1) der äußere Rand der konischen Fläche (3") mit hohem Druck auf der ebenen Fläche (1') aufliegt.

6. Zahnimplantat nach einem der Ansprüche 1 bis 5, **dadurch gekennzeichnet, dass** der obere Abschnitt (1) einen teilweise offenen Ring (5), in dem eine Schraube zur Befestigung des Ringes (5) am oberen Abschnitt (1) eingeschraubt ist, aufweist.

7. Zahnimplantat nach Anspruch 6, **dadurch gekennzeichnet, dass** die Fläche (1") des oberen Abschnittes (1) zur Befestigung des Ringes (5) konisch ausgeformt ist.

8. Zahnimplantat nach Anspruch 6 oder 7, **dadurch gekennzeichnet, dass** der teilweise offene Ring (5) im Bereich seiner Bohrung (5') konvex ausgeführt ist, sodass eine Ausrichtung am chirurgisch getrennten Knochensegment (7) möglich ist.

9. Zahnimplantat nach einem der Ansprüche 1 bis 8, **dadurch gekennzeichnet, dass** der zur Lagefixierung vorgesehene Implantatkopf (3) in Bezug auf seine Formgebung im oberen Ende (3') an die zur prothetischen Versorgung erforderlichen Befestigungsteile angepasst ist.

10. Zahnimplantat nach einem der Ansprüche 1 bis 9, **dadurch gekennzeichnet, dass** die Bohrung zur Aufnahme der Schraube (4) im unteren Abschnitt (2) eine Sackbohrung ist.

11. Zahnimplantat nach einem der Ansprüche 1 bis 9, **dadurch gekennzeichnet, dass** die Bohrung zur Aufnahme der Schraube (4) im unteren Abschnitt (2) eine Durchgangsbohrung ist, in die am Ende ein Stopfen eingepresst oder eingeschweißt ist.

## Claims

1. Dental implant with an implant head and with an enossal part for anchorage in the jawbone that consists of an upper portion (1) and a lower portion (2), these two portions (1, 2) being portable in the axial direction, the lower portion (2) having a tubular extension (2") which juts into a corresponding cylindrical recess in the upper portion (1), and the tubular extension (2") moreover having a female thread into which a screw (4) can be screwed to fix the implant head (3), **characterized in that** the implant head (3) has a cylindrical extension (3"') which fits into the tubular extension (2") of the lower portion (2).

2. Dental implant according to Claim 1, **characterized in that** the tubular extension (2") has a recess (2') for the cylindrical extension (3"') and **in that** the internal diameter of the tubular extension (2") is approximately equal to the internal diameter of the cylindrical extension (3"').

3. Dental implant according to Claim 1 or Claim 2, **characterized in that** a screw (4) extends through at least the entire tubular portion (2").

4. Dental implant according to any one of Claims 1 to 3, **characterized in that** the upper portion (1) and the implant head (3) both have flat faces (3", 1') that come into non-positive connection when the implant head (3) is screwed into the upper portion (1).

5. Dental implant according to any one of Claims 1 to 3, **characterized in that** the upper portion (1) has a flat face (1') and the implant head (3) a slightly conical face (3"), so that the outer rim of the conical face (3") bears on the flat face (1') with high pressure when the implant head (3) is screwed into the upper portion (1).

6. Dental implant according to any one of Claims 1 to 5, **characterized in that** the upper portion (1) has a partly open ring (5) in which a screw is screwed to fasten the ring (5) on the upper portion (1).

7. Dental implant according to Claim 6, **characterized in that** the face (1") of the upper portion (1) for fastening the ring (5) is conically formed.

8. Dental implant according to Claim 6 or Claim 7, **characterized in that** the partly open ring (5) is made convex in the region of its bore (5') to allow alignment on the surgically separated bone-segment (7).

9. Dental implant according to any one of Claims 1 to 8, **characterized in that** the Implant head (3) intended for positional fixing is adapted, in its configuration at the upper end (3'), to the fastenings necessary for prosthetic care.

10. Dental implant according to any one of Claims 1 to 9, **characterized in that** the bore receiving the screw (4) in the lower portion (2) is a blind bore.

11. Dental implant according to any one of Claims 1 to 9, **characterized in that** the bore receiving the screw (4) in the lower portion (2) is a through bore into the end of which a plug is pressed or welded.

## Revendications

1. Implant dentaire comportant une tête d'implant et une partie intra-osseuse à fixer dans l'os de mâchoire, laquelle est composée d'un tronçon supérieur (1) et d'un tronçon inférieur (2), ces deux tronçons (1, 2) étant déplaçables l'un par rapport à l'autre dans la direction axiale, le tronçon inférieur (2) comportant un prolongement tubulaire (2") qui s'avance dans une cavité cylindrique correspondante du tronçon supérieur (1), le prolongement tubulaire (2") comportant en outre un filetage intérieur dans lequel est susceptible d'être vissée une vis (4) pour la fixation de la tête d'implant (3), **caractérisé en ce que** la tête d'implant (3) comporte un prolongement cylindrique (3"') qui s'engage dans le prolongement tubulaire (2") du tronçon inférieur (2).

2. Implant dentaire selon revendication 1, **caractérisé en ce que** le prolongement tubulaire (2") comporte une cavité (2') pour le prolongement cylindrique (3'") et **en ce que** le diamètre intérieur du prolongement tubulaire (2") correspond sensiblement au diamètre intérieur du prolongement cylindrique (3''').

3. Implant dentaire selon la revendication 1 ou 2, **caractérisé en ce qu'**une vis (4) s'étend au moins à travers la totalité du tronçon tubulaire (2").

4. Implant dentaire selon l'une des revendications 1 à 3, **caractérisé en ce que** le tronçon supérieur (1) et la tête d'implant (3) comprennent chacune une surface plane (3", 1') qui entre en liaison à force lors du vissage de la tête d'implant (3) dans le tronçon supérieur (1).

5. Implant dentaire selon l'une des revendications 1 à 3, **caractérisé en ce que** le tronçon supérieur (1) comporte une surface plane (1') et la tête d'implant (3) une surface légèrement conique (3"), de telle sorte que, lors du vissage de la tête d'implant (3) dans le tronçon supérieur (1), la bord extérieur de la surface conique (3") repose avec une importante pression sur la surface plane (1').

6. Implant dentaire selon l'une quelconque des revendications 1 à 5, **caractérisé en ce que** le tronçon supérieur (1) comporte un anneau partiellement ouvert (5) dans lequel est vissée une vis pour la fixation de l'anneau (5) ou au tronçon supérieur (1).

7. Implant dentaire selon la revendication 6, **caractérisé en ce que** la surface (1") du tronçon supérieur (1) pour la fixation de l'anneau (5) est de forme conique obtenue par moulage.

8. Implant dentaire selon la revendication 6 ou 7, **caractérisé en ce que** l'anneau partiellement ouvert (5) est réalisé convexe dans la zone de son alésage (5') de telle sorte qu'un alignement sur le segment d'os (7) tronçonné de manière chirurgicale soit possible.

9. Implant dentaire selon l'une quelconque des revendications 1 à 8, **caractérisé en ce que** la tête d'implant (3), prévue pour le blocage en position, est adaptée en ce qui concerne son profilage dans l'extrémité supérieure (3') aux pièces de fixation nécessaires pour l'équipement prothétique.

10. Implant dentaire selon l'une quelconque des revendications 1 à 9, **caractérisé en ce que** l'alésage pour recevoir la vis (4) dans le tronçon inférieur (2) est un alésage borgne.

11. Implant dentaire selon l'une quelconque des revendications 1 à 9, **caractérisé en ce que** l'alésage pour recevoir la vie (4) dans le tronçon inférieur (2) est un alésage traversant dans lequel, à l'extrémité, est enfoncé ou soudé un bouchon.
